# EUROPÄISCHE PATENTSCHRIFT

(11) **EP 0 412 361 B1**
(45) Veröffentlichungstag und Bekanntmachung des Hinweises auf die Patenterteilung: **22.06.1994**
(21) Anmeldenummer: 90114331.3
(22) Anmeldetag: 26.07.1990
(51) Int. Cl.: C07D 309/18

(54) **Verfahren zur Hestellung von 4-Methylentetrahydropyran**
Process for the preparation of 4-methylenetetrahydropyran
Procédé de préparation de 4-méthylène tétrahydropyranne

(30) Priorität: 08.08.1989 DE 3926170
(43) Veröffentlichungstag der Anmeldung: 13.02.1991
(73) Patentinhaber: BASF Aktiengesellschaft, 67063 Ludwigshafen (DE)
(72) Erfinder: Kuekenhoehner, Thomas, Dr., D-6710 Frankenthal (DE); Spiegler, Wolfgang, Dr., D-6520 Worms 27 (DE); Goetz, Norbert, Dr., D-6520 Worms 1 (DE); Rohr, Wolfgang, Dr., D-6706 Wachenheim (DE)

(56) Entgegenhaltungen:
- US-A- 3 681 263

## Beschreibung

Die vorliegende Erfindung betrifft ein neues Verfahren zur Herstellung von 4-Methylentetrahydropyran (I) 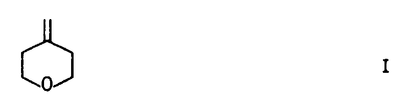
Diese für zahlreiche organische Synthesen, insbesondere im Bereich der Pflanzenschutzmittel wichtige Verbindung entsteht beispielsweise bei der Synthese von Isopren aus Isobuten und Formaldehyd als Nebenprodukt [Chem. Abstr. 77, 134876 (1971)] oder im Gemisch mit dem isomeren 4-Methyl-1-oxa-cyclohex-3-en (I') bei der Thermolyse von 4,4-Dimethyl-1,3-dioxa-cyclohexan [Pet. Chem. USSR (Engl. Transl.) 7 (1967) 92].

Nach der US-A-2 789 996 erhält man sie angeblich auch durch Cyclisierung von 3-Methylenpentan-1,5-diol(II) in Gegenwart von Kaliumhydrogensulfat. Tatsächlich aber bildet sich hierbei nur die isomere Verbindung 4-Methyl-3,6-dihydro-2H-pyran (I') (Sdp. 117°C - 119°C ), wogegen I (Sdp. 108°C - 109°C) nur in Spuren nachgewiesen werden kann, wie eigene Untersuchungen ergaben.

Da diese Verfahren für technische Zwecke offensichtlich wenig oder überhaupt nicht geeignet sind, lag der Erfindung die Herstellung von I in einer einfachen und gezielten Synthese als Aufgabe zugrunde.

Demgemäß wurde ein Verfahren zur Herstellung von 4-Methylentetrahydropyran I 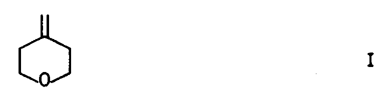
gefunden, welches dadurch gekennzeichnet ist, daß man 3-Methylenpentan-1,5-diol II 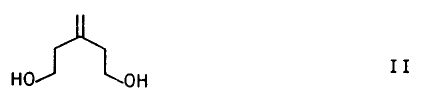
in Gegenwart einer wäßrigen Mineralbase mit einem Sulfonsäurehalogenid(III) umsetzt.

Die Ausgangsverbindung, das 3-Methylenpentan-1,5-diol(II) ist literaturbekannt und durch Erhitzen von Paraformaldehyd mit Isobuten [J. Amer. Chem. Soc. 77, 4,666 - 4,668 (1955)] oder mit 3-Methylbut-3-en-1-ol (US-A-2 789 996) erhältlich.

Die Eignung der Sulfonsäurehalogenide(III) ist nach den bisherigen Beobachtungen nicht von der Art des Säurerestes abhängig, so daß man grundsätzlich beliebige Verbindungen dieser Art einsetzen kann. Aus wirtschaftlichen Gründen bevorzugt man aber die Chloride von C₁-C₄-Alkansulfonsäuren wie Methansulfochlorid und Ethansulfochlorid sowie die Chloride aromatischer Sulfonsäuren wie Benzolsulfochlorid, p-Toluolsulfochlorid, p-Chlorbenzolsulfochlorid und p-Brombenzolsulfochlorid. Die Menge an Sulfonsäurechlorid ist nicht kritisch, jedoch empfiehlt es sich zur Erzielung eines vollständigen Umsatzes mindestens 1 mol, vorzugsweise bis zu 2 mol pro Mol II einzusetzen.

Die Mineralbasen haben die Funktion, den bei der Reaktion freiwerdenden Halogenwasserstoff zu binden. In Betracht kommen daher vor allem Alkali- und Erdalkalimetallhydroxide und -oxide wie Lithium-, Natrium-, Kalium- und Calciumhydroxid sowie Alkalimetall- und Erdalkalimetallcarbonate wie Natrium-, Kalium- oder Calciumcarbonat. Vorzugsweise verwendet man Natrium- oder Kaliumhydroxid.

Die Konzentration der Mineralbasen liegt vorzugsweise zwischen 1 und 50 Gew.-%. Ihre Menge sollte zur Erzielung eines vollständigen Umsatzes mindestens äquimolar zur Menge des Diols II sein, jedoch empfiehlt sich in der Regel ein Molverhältnis von Base: II von 2 : 1 bis 8 : 1.

Da es sich um eine Zweiphasen-Reaktion handelt, empfiehlt sich in aller Regel die Mitverwendung eines in Wasser unlöslichen oder schwer löslichen Lösungsmittels, um das Volumen der organischen Phase zu vergrößern.

Als Lösungsmittel eignen sich beispielsweise aliphatische und aromatische Kohlenwasserstoffe wie Pentan, Hexan, Heptan, Cyclohexan, Benzin, Petrolether, Ligroin, Benzol, Toluol, Xylol, halogenierte aliphatische und aromatische Kohlenwasserstoffe wie Methylenchlorid, Ethylenchlorid, Tetrachlorkohlenstoff, Chlorbenzol und o-Dichlorbenzol sowie Ether wie Diethyl- und Dibutylether, Glykoldimethylether, Methyl-tert.-butylether und Diglykoldimethylether, Tetrahydrofuran und Dioxan. Besonders einfach gestaltet sich das Verfahren, wennn man das Verfahrensprodukt I als Lösungsmittel verwendet.

Die Menge des Lösungsmittels wird zweckmäßigerweise so bemessen, daß das Volumen der organischen Phase 10 bis 200 % der wäßrigen Phase beträgt.

Da die Reaktanden vornehmlich an der Phasengrenzfläche miteinander reagieren, kann die Mitverwendung eines Phasentransferkatalysators von Vorteil sein. Allerdings ist hierbei zu berücksichtigen, daß durch die Phasentransferkatalyse auch die unerwünschte Verseifung des Sulfochlorides begünstigt werden kann. Als Phasentransferkatalysatoren eignen sich die hierfür gebräuchlichen quartären Ammonium- und Phosphoniumsalze, beispielsweise Triethylbenzylammoniumbromid.

Die Reaktionstechnik der Phasentransferkatalyse, die sich ohne weiteres auf das erfindungsgemäße Verfahren übertragen läßt, ist allgemein bekannt, so daß sich nähere Angaben hierzu erübrigen.

Vorzugsweise nimmt man die Umsetzung bei 20°C bis 100°C, besonders bei 80°C - 100°C vor, wobei sich das Erhitzen unter Rückflußkühlung unter Verwendung eines entsprechend siedenden Lösungsmittels empfiehlt.

Normalerweise arbeitet man unter Atmosphärendruck. Geringerer Druck, etwa im Bereich von 0,5 bis 1 bar, kann zweckmäßig sein, wenn man das Verfahrensprodukt möglichst schnell aus der Reaktionsmischung mit einem hochsiedenden Lösungsmittel entfernen will, und höherer Druck, etwa bis zu 5 bar, kann im Falle tiefsiedender Lösungsmittel erforderlich sein.

Methodisch bietet das erfindungsgemäße Verfahren keine Besonderheiten, d. h. es läßt sich nach den üblichen Techniken diskontinuierlich oder kontinuierlich ausführen. Gut bewährt hat es sich, eine Mischung aus der wäßrigen Mineralbase, der organischen Lösung des Diols II und gegebenenfalls dem Phasentransferkatalysator vorzulegen und hierzu bei Reaktionstemperatur das Sulfochlorid oder eine Lösung des Sulfochlorids zu geben.

Auch die Aufarbeitung kann man wie üblich vornehmen. Trennt man die organische Phase ab, neutralisiert, wäscht und trocknet sie, so ist allerdings darauf zu achten, daß bei der anschließenden Destillation Zersetzungsreaktionen auftreten können. Es ist daher für den Fall eines tiefsiedenden Lösungsmittels zu bevorzugen, zunächst dieses abzudestillieren und danach das Verfahrensprodukt als Hetero-Azeotrop mit Wasser zu gewinnen.

Man erhält das 4-Methylentetrahydropyran in Ausbeuten zwischen etwa 60 und 80 %.

### Beispiele 1 bis 6

### Herstellung von 4-Methylentetrahydropyran (I)

Eine Mischung aus 348 g (3 mol) 3-Methylenpetan-1,5-diol, 2670 g 30 gew.-%iger Natronlauge (≙ 20 mol NaOH) und 100 ml eines organischen Lösungsmittels wurde bei der Rückflußtemperatur T°C im Laufe einer Stunde mit 635 g (3,6 mol) Benzolsulfonsäurechlorid versetzt, noch 15 min bei T°C gehalten und danach wie üblich destillativ auf das 4-Methylentetrahydropyran aufgearbeitet, wobei dieses als Hetero-Azeotrop mit Wasser gewonnen wurde. Die Einzelheiten dieser Versuche sowie deren Ergebnisse sind der nachstehenden Tabelle zu entnehmen.

| Beispiel | Lösungsmittel | T [°C] | Ausbeute an I [%] |
|---|---|---|---|
| 1 | I | 95 - 105 | 75 |
| 2 | Tetrahydrofuran | 60 - 70 | 67 |
| 3 | Toluol | 90 - 100 | 63 |
| 4 | Methyl-tert.-butylether | 55 - 65 | 75 |
| 5 | Cyclohexan | 75 - 85 | 66 |
| 6* | Methyl-tert.-butylether | 55 - 65 | 78 |

| | | | |
|---|---|---|---|
| * 500 ml Lösungsmittel (8 mol Natronlauge) | | | |

## Patentansprüche

1. Verfahren zur Herstellung von 4-Methylentetrahydropyran (I) 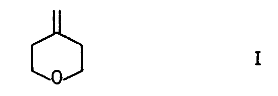 dadurch gekennzeichnet, daß man 3-Methylenpentan-1,5-diol (II) 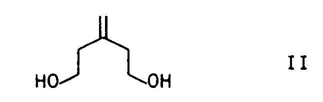 in Gegenwart einer wäßrigen Mineralbase mit einem Sulfonsäurehalogenid(III) umsetzt.

2. Verfahren nach Anspruch 1, dadurch gekennzeichnet, daß man die Umsetzung in Gegenwart eines in Wasser nicht oder nur schwer löslichen Lösungsmittels vornimmt.

3. Verfahren nach den Ansprüchen 1 und 2, dadurch gekennzeichnet, daß man bei der Umsetzung einen Phasentransferkatalysator mitverwendet.

4. Verfahren nach den Ansprüchen 1 bis 3, dadurch gekennzeichnet, daß man als Sulfonsäurehalogenid(III) ein C₁-C₄-Alkylsulfonsäurechlorid oder ein aromatisches Sulfonsäurechlorid verwendet.

## Claims

1. A process for preparing 4-methylenetetrahydropyran (I) 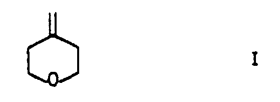 which comprises reacting 3-methylenepentane-1-5-diol (II) 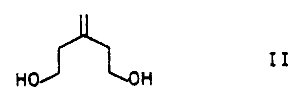 with a sulfonyl halide (III) in the presence of an aqueous inorganic base.

2. A process as claimed in claim 1, wherein the reaction is carried out in the presence of a solvent which is insoluble or only slightly soluble in water.

3. A process as claimed in claims 1 and 2, wherein a phase-transfer catalyst is additionally used during the reaction.

4. A process as claimed in claims 1 to 3, wherein the sulfonyl halide (III) used is a C₁-C₄-alkylsulfonyl chloride or an aromatic sulfonyl chloride.

## Revendications

1. Procédé de préparation de 4-méthylène-tétrahydropyranne (I) 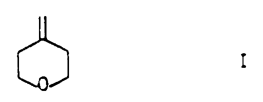 caractérisé en ce qu'on fait réagir du 3-méthylène-pentane-1,5-diol (II) 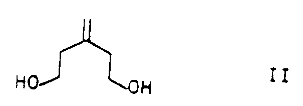 en présence d'une base minérale aqueuse, avec un halogenure (III) d'acide sulfonique.

2. Procédé selon la revendication 1, caractérisé en ce qu'on conduit la réaction en présence d'un solvant qui est insoluble ou n'est que difficilement soluble dans l'eau.

3. Procédé selon les revendications 1 et 2, caractérisé en ce qu'on utilise simultanément, dans la réaction, un catalyseur de transfert de phase.

4. Procédé selon l'une quelconque des revendications 1 à 3, caractérisé en ce qu'on utilise, comme halogénure (III) d'acide sulfonique, un chlorure d'acide (alkyl en C₁-C₄)sulfonique ou un chlorure d'acide sulfonique aromatique.
